# EUROPEAN PATENT APPLICATION

(11) **EP 3 598 895 A1**
(43) Date of publication of application: **29.01.2020**
(21) Application number: 18382550.4
(22) Date of filing: 23.07.2018
(51) Int. Cl.: A01N 1/02, C12N 5/00, C12N 5/073

(54) **AN IN VITRO METHOD FOR FREEZING MAMMALIAN EMBRYOS**

(71) Applicant: Servicio Regional de Investigación y Desarrollo Agroalimentario (Serida), 33300 Villaviciosa Asturias (ES)
(72) Inventor: GÓMEZ PIÑEIRO, Enrique, 33394 Deva (Gijón) (ES); CARROCERA COSTA, Susana, 33394 Deva (Gijón) (ES); MARTÍN GONZÁLEZ, David, 33394 Deva (Gijón) (ES)
(74) Representative: Pons

(57) **Abstract**

The invention relates to an *in vitro* method for freezing mammalian embryos, comprising culturing at least one mammalian embryo for a period between 4 and 8 days, next culturing individually mammalian embryos in a medium without protein, and finally freezing the embryos in liquid nitrogen in the presence of a freezing composition comprising ethylene glycol, phosphate buffered saline and synthetic cryoprotectant CRYO3. The invention further relates to a method for reproducing mammals using the *in vitro* method for freezing mammalian embryos herein disclosed, increasing the births rate when the embryos are transferred to the recipients.

## Description

The invention relates to an *in vitro* method for freezing mammalian embryos, comprising culturing individually mammalian embryos in a medium without protein, and freezing the embryos in liquid nitrogen in the presence of a freezing composition comprising ethylene glycol, phosphate buffered saline and synthetic cryoprotectant CRYO3. Thus, the present invention relates to the field of embryo cryopreservation and particularly relates to an in-vitro embryo culture solution for increasing birth rates when the embryos are transferred to the recipients. Thus, the invention further relates to a method for reproducing mammals.

### BACKGROUND ART

Cryopreservation is essential in assisted reproductive technology, as it allows embryo storage for very long periods, successful commercial worldwide exchanges, and it is necessary in case of recipient shortage or embryo surplus. Two main cryopreservation procedures are available for bovine embryos: freezing/thawing (F/T), and vitrification/warming (V/W). Therefore, improving survival after F/T and V/W are major objectives in cattle embryo technology.

F/T and V/W have each particular advantages and disadvantages. Thus, V/W is simple, with no expensive equipment required; cheap and, when embryos are few, no time consuming. However, V/W requires well trained skills, direct transfer is rather a challenge, and some vitrification procedures are not compliant with sanitary requirements. On its side, F/T requires expensive equipment but allows faster management when embryos are many, and these techniques fulfill sanitary requirements. A desirable step that facilitates on farm application of cryopreserved embryos is direct transfer (DT) in straw. Within F/T, DT has greatly simplified post-thawing rehydration with *in vivo* embryos.

V/W techniques have been generally described to perform better than F/T with in vitro produced (IVP) embryos. However, this notion lies on comparisons between V/W and F/T mainly based on in vitro experiments. In contrast, experiments that incorporate embryo transfer (ET) to compare F/T vs. V/W are scarce and constrained by embryo selection and discard prior to ET, low numbers of ETs or circumscribed to cohorts of micromanipulated embryos. However, upon acceptance of any kind of embryo cryopreservation must reduce pregnancy and/or birth rates, it is surprising to confirm that a number of reports performed with high numbers of ETs with IVP embryos that analyzed F/T vs. fresh embryos do not find such reductions or the decrease in pregnancy and birth rates for F/T embryos is not striking, being in the survival range observed for V/W embryos vs. fresh embryos. Together with the above, cattle breeding industries use F/T technology with IVP embryos for commercial exchanges (personal observation). This suggests that recent improvements led to F/T technology with efficacy levels comparable to V/W within IVP embryos. Such information, nevertheless, was not publicly available until a recent work from Sanches et al. 2016. Theriogenology, 85: 1147-1151. These authors obtained high pregnancy rates (PR) with an F/T procedure with DT of IVP embryos. The technique described is a modification of a classical ethylene-glycol (EG)-based technique [Voelkel & Hu, et al. 1992.Theriogenology, 37: 23-37], simple and performed on N=800 ET operations (fresh, V/W and F/T) in a unique herd with parous recipients. Although non-significant, F/T pregnancy rates were above those of V/W, both below the PR reported with fresh embryos. Collectively, the above reports are not supportive of V/W performing better than F/T when IVP embryos undergo development to term upon transfer to recipients.

*In vitro* embryo production is a major component of cattle breeding. Often, more embryos are produced than are implanted. Although over 30% of conception rates have recently been achieved in the embryo transfer (ET) of vitrified in vitro produced (IVP) embryos, the complexity of recovery of these embryos after vitrification remains an obstacle for commercial applications of this technique. There is a need for novel, efficient protocols for mammalian embryo cryopreservation, mainly, bovine embryo.

### DETAILED DESCRIPTION OF THE INVENTION

The inventors of the present invention have discovered that if bovine embryos are individually cultured for a short period in the absence of serum or protein, and next the embryos are frozen with a freezing composition comprising ethylene glycol (EG), phosphate buffered saline (PBS) and synthetic cryoprotectant CRYO3, the miscarriage of the embryos is surprisingly reduced and higher birth rates are achieved when embryos are transferred to recipients.

Thus, the present invention provides an efficient chemically defined freezing/thawing (F/T) system for IVP embryos, based on an embryo culture step without protein, replacement of BSA and/or serum by a chemical supplement such as CRYO3 in cryoprotectant solutions, and a modification of the straw cryoprotectant distribution. These combinations yield high pregnancy and birth rates. The present disclosure demonstrates the effectiveness of improved cryopreserving technology for freezing and thawing embryos.

Thus, in an aspect, the present invention relates to an *in vitro* method for freezing mammalian embryos, hereinafter "method of the invention", comprising
(a) culturing at least one mammalian embryo for a period between 4 and 8 days,
(b) culturing individually the mammalian embryo of step (a) in the presence of a composition which does not comprise serum or proteins, and
(c) freezing the embryos from step (b) in liquid nitrogen (LN) in the presence of a freezing composition comprising ethylene glycol (EG), phosphate buffered saline (PBS) and synthetic cryoprotectant CRYO3.

As used herein, the term "embryo" refers to the early period of development from the zygote to the blastocyst stage, including the blastocyst expansion and hatching, which leads to expanded blastocysts and hatched blastocysts. Any embryo can be used in the method of the invention no matter its stage. The embryo to be frozen may be, for example, at zygote, morula, early blastocyst, blastocyst, expanded blastocyst, trophoblast or embryoblast stage.

As used herein, the term "zygote" refers to a cell formed when two gamete cells are joined by means of sexual reproduction. It is the earliest developmental stage of the embryo. A zygote is synthesized from the union of two gametes, and represents the first stage in a unique organism's development. Zygotes are produced by fertilization between two haploid cells -an ovum (female gamete) and a sperm cell (male gamete)- which combine to form the single diploid cell.

As used herein, the term "morula" refers to a stage of embryonic development. The morula, an early stage embryo which consists of a ball of cells (called blastomeres) contained within the zona pellucida, is produced from the single-celled zygote by a series of cleavages. Through cellular differentiation and cavitation, the morula gives rise to the blastocyst. Once a fluid-filled cavity begins to open up in the morula, the blastocyst stage of embryonic development starts. During blastocyst formation, the morula's cells differentiate into an inner cell mass growing inside the blastocoel and trophoblast cells forming the outer blastocoel membrane.

As used herein, the term "blastocyst" refers to a structure formed in the early development of mammals. It possesses an inner cell mass (ICM) which subsequently forms the embryo itself. The trophoblast is the outer layer of cells of the blastocyst. This layer surrounds an inner cell mass (which is a source of embryonic stem cells) and a fluid-filled cavity known as the blastocoele. The trophoblast gives rise to the placenta. The use of blastocysts for in-vitro fertilization (IVF) involves culturing a fertilized egg before implanting it into a bovine uterus.

After blastocyst formation, the ungulate embryo (i.e., in cattle, pigs and sheep) undergoes a period of elongation prior to implantation on the uterine wall. This elongation period does not occur in primate and mouse embryos. In some embodiments, the early blastocyst stage is characterized by a cavity which is just beginning to form and blastocyst cells are not yet distinguishable. In some embodiments, the expanded blastocyst stage is characterized by a fully formed cavity, increased size and thinner zona pellucida. Likewise, any embryo coming from any mammalian can be used in the method of the invention. Examples of mammals include, without limiting to, primates, cattle, sheep, pigs, goats, horses, mules, cats, rabbits, rats, and mice. In a particular embodiment of the method of the invention, the mammalian embryo is an ungulate embryo, preferably, a bovine embryo.

As used herein, the term "ungulate" refers to a diverse group of large mammals that includes equines, bovines/cattle, pigs, goats, buffalo, sheep, giraffes, camels, deer, and hippopotamuses. Most terrestrial ungulates use the tips of their toes, usually hoofed, to sustain their whole body weight while moving. In some embodiments, the term means, roughly, "being hoofed" or "hoofed animal".

The present method can include the use of embryos produced by various *in vitro* techniques including nuclear transfer, embryo splitting, fertilization of *in vitro* matured oocytes, *in vitro* fertilization of oocytes, *in vitro* culture of fertilized oocytes, gene transfer of exogenous DNA to an embryo, and artificial insemination. In a particular embodiment of the method of the invention, the bovine embryos are those obtained by *in vivo* or *in vitro* fertilization.

Development of embryo transfer (ET) technology allows producers to obtain multiple progeny from genetically superior females. Fertilized embryos can be recovered from females (also called embryo donors) of superior genetic merit by surgical or nonsurgical techniques. The genetically superior embryos are then transferred to females (also called embryo recipients) of lesser genetic merit. In cattle, efficient techniques can recover fertilized embryos without surgery, but only one or sometimes two embryos are produced during each normal reproductive cycle. To increase the number of embryos that can be recovered from genetically superior females, the embryo donor is treated with a hormone regimen to induce multiple ovulations, or superovulation.

*In vitro* production of embryos is a three-step process involving oocyte maturation, oocyte fertilization and in vitro culture. Only 30-40% of such oocytes reach the blastocyst stage, at which they can be transferred to a recipient or frozen for future use. The quality of the oocyte can dramatically impact the proportion of immature oocytes that form blastocysts while the post-fertilization culture environment has a major influence on the quality of the blastocyst. Assessment of embryo quality is a challenge. Morphological assessment is at present the most popular method for embryo selection prior to transfer. Other non-invasive assessment methods include the timing of the first cleavage division which has been linked to developmental ability. Quantitative examination of gene expression is an additional valuable tool to assess the viability of cultured embryos. A substantial amount of evidence exists to demonstrate that the culture conditions, to which the embryo is exposed, particularly in the post-fertilization period, can have perturbing effects on the pattern of gene expression in the embryo with potentially important long-term consequences.

Thus, in a first step [step (a)], the method of the invention comprises culturing at least one mammalian embryo, i.e. either individually or in groups, for a period between 4 and 8 days, preferably between 5 and 7 days, more preferably 6 days. The period of incubation depends on the embryo stage at which it is desired to freeze the embryo. For example, in a particular embodiment, if the embryo/s is/are cultured until Day-6 post-insemination time (PI), excellent and good quality (grade 1 and grade 2) morulae and early blastocysts are obtained. Then, these embryos will be selected and used in step b) of the method of the invention. Embryo culture until blastocyst stages later than early blastocyst confers a significant increase in live birth rate per embryo transfer.

As used herein, the term "embryo" has been defined above. The culture of step (a) can be carried out using any medium of the state of the art suitable for the culture of embryos. The conditions of culture (pH, temperature, humidity, osmolarity, amount of gas, etc.) are those usually used in the state of the art. In some embodiments, the presence or amount of a gas is selected from the group consisting of O₂ and CO₂. In a particular embodiment of the invention, in vitro culture was carried out at 38.7 °C, 5% CO2, 5% O2, 90% N2, and saturated humidity.

The embryo cells may be placed in an animal cell culture medium normally appropriate for maintaining the health and viability of animal cells. The preferred culture medium for a given application, e.g., for cells of a particular species and a particular development stage, will be known to one skilled in the art. Examples of known culture media which may be used in step (a) for the purpose of the present invention include Ham's F-10+10% fetal calf serum (Ham's), Tissue Culture Medium-199+10% fetal calf serum (TCM-199), G1/G2 medium, potassium simple optimized medium (KSOM) embryo, Eagle's and Whitten's media, or any other media compatible with the continued viability of the embryos, preferably, bovine. These listed media, their components and their methods of use are well known to those skilled in this art, as are a variety of other media. A medium will be considered suitable for the present invention if it is known to maintain embryonic cells in culture in a healthy and viable condition. In a particular embodiment, the culture medium used in step a) of the method of the invention is synthetic oviduct fluid (SOF) medium. In a more particular embodiment, the culture medium used in step a) of the method of the invention is synthetic oviduct fluid (SOF) medium comprising amino acids (such as, but not limited to, BME amino acids solution, and MEM non-essential amino acids solution), citrate, myo-inositol, and Bovine serum albumin (BSA) with or without fetal calf serum (FCS).

The culture medium may be conditioned for the culture of embryos by culture of epithelial cells in that medium, followed by removal of the epithelial cells prior to step (b) of the method of the invention. While 20 hours of culture of the epithelial cells in the medium has been found sufficient to condition the medium for embryo development, it is to be understood that wide variations in this time period are possible, depending on type of medium, concentration and vitality of epithelial cells and similar factors. The parameters of the conditioning step can be readily determined for the selected media and conditions. The epithelial cells may be completely removed from the medium, by centrifugation, filtration or other technique prior to culturing the embryos as defined in step (b). It may also be possible to separately synthesize or generate the embryotrophic factor and add it to the medium directly.

The culture of the embryos in step (a) lasts between 4 and 8 days. After these days, the embryos are cultured individually as defined in step (b) of the method of the invention.

Next, in a second step [step (b)], the method of the invention comprises culturing individually the mammalian embryos obtained in step a) in the presence of a composition which does not comprise proteins. The culture of the individually mammalian embryos lasts between 15 and 45 hours, preferably, between 20 and 40 hours, more preferably, 24, 28, 32 or 36 hours. During this step, the lipid granule contents of embryos decrease, and a gene expression profile of lipid breakdown occurs, which, without being bound by any theory, is thought to improve ability to survive cryopreservation.

Any embryo culture medium can be used in step b) of the method of the invention as long as it does not comprise proteins.

As used herein, the term "protein" generally refers to a linear chain of amino acid residues joined together with peptide bonds comprising more than 3 amino acid residues. The proteins can be from any origin. It will be understood that the term "peptide bond" is known to the person skilled in the art. As used herein, an "amino acid residue" refers to any naturally occurring amino acid, any amino acid derivative or any amino acid mimic known in the art. The term "amino acid residue" encompasses both L- and D-amino acid residues.

Examples of proteins usually used in the embryo culture medium but, in the present invention, are not present include, without limiting to, Bovine Serum Albumin (BSA) at different purity levels, such as found in commercial preparations; Essentially Fatty-acid free BSA; globulin-free BSA; Globulin extracts; Egg yolk extracts and derivatives; Fetal calf serum; Calf serum; Steer Serum; Cow serum; superovulated cow serum; filtered serum; charcoal treated serum; proteins or high molecular weight extracts of serum and derivatives, oviduct fluid and uterine fluid; recombinant proteins, particularly recombinant albumins, including human and bovine albumin and any other protein. In a particular embodiment of the method of the invention, the composition of step (b) for culturing the embryos does not comprise bovine serum albumin (BSA) nor fetal calf serum (FCS).

Thus, in the context of the present invention, compositions which cannot be used for culturing the embryos according to step b) of the method of the invention include, without limiting to, blood plasma, blood serum or blood serum derivatives; uterine fluid or its derivatives; and/or natural oviductal fluid from genital truck or its derivatives. However, the embryo culture medium of step b) can contain free o di-peptide forming amino-acids.

As used herein, the term "plasma" or "blood plasma" refers to the yellowish coloured liquid component of blood that normally holds the blood cells in whole blood in suspension; this makes plasma the extracellular matrix of blood cells. Blood plasma is separated from the blood by spinning a tube of fresh blood containing an anticoagulant in a centrifuge until the blood cells fall to the bottom of the tube. The blood plasma is then poured or drawn off.

As used herein, the term "serum" or "whey" or "blood serum" refers to both the clear yellowish fluid obtained upon separating whole blood into its solid and liquid components after it has been allowed to clot, and the watery fluid from animal tissue, such as that found in edema. Thus, the serum is the component that is neither a blood cell (serum does not contain white or red blood cells) nor a clotting factor; it is the blood plasma not including the fibrinogens. Serum includes all proteins not used in blood clotting (coagulation) and all the electrolytes, antibodies, antigens, hormones, and any exogenous substances (e.g., drugs and microorganisms).

As used herein, the term "uterine fluid" refers to fluid coming from the uterine cavity, wherein said fluid has not been treated or modified for deleting any of the components naturally present in this kind of fluids.

As used herein, the term "natural oviductal fluid from genital tract" or "oviduct fluid" refers to the fluid coming from the oviduct. In the present description, "oviduct", "Fallopian tube", "uterine tube" and "tube" are used interchangeably.

Culture of embryos can either be performed in an artificial culture medium as long as it does not comprise proteins. With artificial culture medium, there can either be the same culture medium throughout the period, or a sequential system can be used, in which the embryo is sequentially placed in different media. Artificial embryo culture media basically contain glucose, pyruvate, and energy-providing components, but the addition of amino acids (free o di-peptide forming amino-acids), nucleotides, vitamins, and cholesterol improve the performance of embryonic growth and development. Methods to permit dynamic embryo culture with fluid flow and embryo movement are also available. In a particular embodiment of the method of the invention, the composition for culturing the embryos in step (b) is synthetic oviduct fluid medium at 5% O2. Use of low oxygen concentrations of 5% rather than about 20% in the atmosphere has been shown to increase live birth rate to a relative probability of 1.24, without any evidence of increased risk for miscarriages or congenital abnormalities.

Once the mammalian embryos have been individually cultured as defined in step (b), the method of the invention comprises freezing the embryos in liquid nitrogen (LN) in the presence of a freezing composition (or freezing solution) comprising ethylene glycol (EG), phosphate buffered saline (PBS) and synthetic cryoprotectant CRYO3 [step c) of the method of invention]. In a particular embodiment, the freezing composition wherein the embryos are loaded comprises PBS, 1.5M EG and 20% CRYO3. Before immersing the embryos into the freezing composition, these may be examined in order to detect defective embryos.

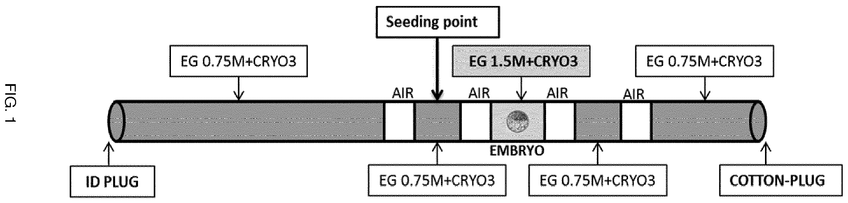

Once the embryos have been immersed into freezing composition, these are loaded into French straws or insemination straws. The straws can be plastic straws of very fine diameter such as are conventionally used in artificial insemination techniques. Such straws are familiar to those skilled in the art and can, for example, be what are known to those in the artificial insemination industry as "French straws" such as those marketed by I.M.V. of L' Aigle, France. Such straws can be purchased in either colored or clear varieties and come in various sizes including, for example, a 0.5 cc capacity straw and a "fine" straw having a capacity of 0.25 cc. Many of these straws include a sealing powder, or plug, which is made of a dry porous material which seals once it becomes moist. Such straws can be used with artificial insemination guns which effectively push the sealing plug through the interior diameter of the straw thereby forcing all the liquid material contained in the straw out its open end. The straws are prepared comprising layers of freezing medium separated by air barriers, wherein the middle layer comprises the freezing composition comprising the embryos obtained in step a) of the method. Thus, in a particular embodiment of the method of the invention, the embryos of step (b) are loaded into a French straw between two columns comprising PBS, 0.75M EG and 20% CRYO3, and two further columns comprising PBS, 0.75M EG and 20% CRYO3 in turn separated by air (see Figure 1). Sealant/identification rods are placed on the open end of the straws which are then frozen by controlled-rate freezing temperatures immersion into liquid nitrogen.

Methods and conditions for freezing embryos are known in the state of the art. In a particular embodiment, the embryos are maintained at -6°C for 2 minutes and seeded once with supercooled forceps only in the upper column adjacent to that contained the embryo. Straws remained for 8 further minutes at -6°C and were subsequently dehydrated at -0.5°C/minutes up to -32°C. Ten to fifteen minutes after reaching this temperature, the straws were stored in LN2 until use.

As used herein, the term "receptacle" refers to a device for containing one or more cryopreserved embryos. The receptacle may contain a series of regions, or chambers, for holding: air, thawing solution, or embryo in cryopreservation solution. In some embodiments, the receptacle is or comprises a straw.

Embryos can be thawed by removing the straw from the liquid nitrogen and immediately immersing it into a room temperature water bath for five to 10 seconds, with both ends of the straw still sealed, and emptying the contents into a dish of dilution medium for five minutes. In a particular embodiment, for thawing, the embryos were held for 10 seconds on air, and then 30 seconds at 35 °C in a water bath. The straws were carefully dried with disposable wipes humidified with 70% ethanol.

In view of the foregoing, in another aspect, the present invention also encompasses an *in vitro* use of a freezing composition comprising ethylene glycol (EG), phosphate buffered saline (PBS) and synthetic cryoprotectant CRYO3 for freezing mammal, preferably bovine, embryos cultured in the absence of proteins. Hereinafter called "use of the invention". The terms and expressions used in the present aspect of the invention has been defined for the first method of the invention, and such definitions as well as its particular embodiments are also applicable to the present inventive aspect.

As explained at the beginning of the present description, the method of the invention allows preserving embryos for being used in IVF techniques or in embryos transfer, reducing the miscarriage of the embryos and achieving higher births rates when they are transferred to the female recipients.

Thus, in another aspect, the present invention relates to an *in vitro* method for obtaining mammalian embryos with high birth rates after embryo transfer comprising, hereinafter second method of the invention, comprising:
(a) Freezing an embryo from the mammal to be reproduced by a method according to the method of the invention as explained above in the present description, and
(b) Thawing of the frozen embryos from step (a).

As used herein, the expression "mammalian embryos with high birth rates after embryo transfer" refer to embryos which, after being transferred to a female recipient, have higher birth rates than embryos obtained by protein-containing methods.

Step (a) of the second method of the invention has been explained in previous paragraphs for the first method of the invention together with the particular embodiments thereof. They are applicable to the present inventive aspect. Next, the second method of the invention comprises thawing the frozen embryos from step (a).

Techniques for thawing frozen embryos are widely known in the state of the art. As explained above, embryos can be recovered by removing the straw from the liquid nitrogen and immediately immersing it into a room temperature water bath for five to ten seconds, unsealing both ends of the straw, and emptying the contents into a dish of dilution medium for five minutes. Following dilution, rehydration occurs when the embryos are placed into a volume of rehydration/recovery medium for fifteen minutes. After the proper incubation time, the embryos can be implanted into the recipient females for development into offspring. High yields are obtained by use of media all containing the same ion concentration, short equilibration times, and proper straw loading technique.

However, in a particular embodiment of the second aspect of the invention, thawing of the frozen embryos comprises exposing the embryos to room temperature during a time between 9 to 11 seconds and next immersing them into water at a temperature between 33 to 37°C during 28 to 32 seconds. In a more particular embodiment, melting of the frozen embryos comprises exposing the embryos to room temperature during 10 seconds and next immersing them into water at 35°C during 30 seconds.

The second method of the invention can be applied to any mammal. Examples of mammals include, without limiting to, primates, cattle, sheep, pigs, goats, horses, mules, cats, rabbits, rats, and mice. In a particular embodiment of the second method of the invention, the mammal is a bovine.

As the skilled person knowns, once embryos are thawed, they are transferred to a female recipient. Embryo transfer refers to a step in the process of assisted reproduction in which embryos are placed into the uterus of a female with the aim to establish a pregnancy. Depending on the mammalian to be used as recipient, the conditions and protocol of the transfer may vary. In any case, as the skilled person knows, in order to successfully transfer the embryos, the oestrus cycle of the female recipient (i.e., days after ovulation) has to be synchronised with the embryonic age to facilitate the pregnancy establishment and maintenance to term.

Thus, in another aspect, the present invention relates to a method for reproducing mammals comprising:
(a) Freezing an embryo from the mammal to be reproduced by a method according to the method of the invention as explained above in the present description,
(b) Thawing of the frozen embryos from step (a), and
(c) Transferring the thawed embryos to female recipients.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

Figure 1. In-straw distribution of cryoprotectant solutions and embryo for freezing and direct transfer. EG: ethylene-glycol. CRYO3: macromolecular replacement.

### EXAMPLES

### Efficient direct transfer to recipients of frozen/thawed bovine embryos cultured in vitro

### MATERIALS AND METHODS

All experimental procedures were approved by the Animal Research Ethics Committee of SERIDA (PROAE 26-2016; Decision of July 25, 2016 of Consejeria de Medio Rural y Recursos Naturales), in accordance with the European Community Directive 86/609/EC.

All reagents were purchased from SIGMA (Madrid, Spain) unless otherwise stated. General procedures for *in vitro* embryo production have been described (Muñoz M, et al. 2014. J Dairy Sci. 97(9):5497-507; Gómez E, et al. 2008. Reprod Fertil Dev. 20(8): 884-91). The following are descriptions in brief.

### Oocyte collection and in vitro maturation (IVM)

Ovaries were collected from slaughtered cows (Matadero de Guarnizo, Cantabria, Spain). Ovaries were transported to the laboratory in saline with penicillin 100 IU/mL and streptomycin sulfate 100 µg/mL, and kept at 25°C to 30°C during collection and transportation.

Antral follicles (3-8 milimeters in diameter) were aspirated with an 18-g needle connected to a syringe, and transferred to holding medium (HM) (TCM199 (Invitrogen, Barcelona, Spain), 25 mM HEPES and 0.4 mg/mL BSA). Good-quality oocytes with more than three layers of compact cumulus cells and homogenous cytoplasm were selected for *in vitro* maturation (IVM). The cumulus-oocyte complexes (COCs) were rinsed three times in HM. Selected COCs were washed three times in maturation medium (MM) consisting of TCM199 NaHCO3 (2.2 mg/mL) supplemented with 10% (v/v) FCS (F4135), 1.5 µg/mL of porcine FSH-LH (Stimufol; ULg FMV, Liege, Belgium) and 1 µg/mL 17 β-estradiol. COCs were transferred (n=30-50) along with 500 µL of IVM medium into each well of a four-well dish and cultured for 22 to 24 hours at 38.7°C, 5% CO2 and high humidity.

### In vitro fertilization (IVF)

Commercial frozen sperm from Asturiana de los Valles bulls (N=2) and Holstein (N=3) bulls with proven fertility were thawed and used for IVF (Day 0). Motile sperm was obtained by a swim-up procedure. Thawed semen was added to a tube with 1mL of pre-equilibrated Sperm-TALP (Tyrode's albumin lactate pyruvate). After 1 hour incubation, the supernatant upper layer with motile sperm was recovered. Sperm was centrifuged for 7 minutes at 200x g and the supernatant was aspirated. COCs were washed twice in HM and placed in four-well culture dishes containing pre-equilibrated fertilization medium (Fert-TALP) with heparin (10 µg/mL; Calbiochem, La Jolla, CA, USA). Spermatozoa were added at a concentration of 2 x 106 cells/mL in 500 µL of medium per well, with a maximum of 50 COCs. IVF was accomplished by incubating oocytes and sperm cells together for 18 to 20 hours at 38.7°C in 5% CO2 atmosphere with saturated humidity.

### In vitro culture (IVC)

Cumulus cells were detached using a vortex and fertilized oocytes were cultured in modified synthetic oviduct fluid (mSOF) containing amino acids (BME amino acids solution, 45 µL/mL and MEM non-essential amino acids solution, 3.3 µL/mL), citrate (0.1 µg/mL), myo-inositol (0.5 µg/mL), and BSA (6 mg/mL) with 0.1% (v/v) FCS (SIGMA F4135) or without FCS (Murillo A, et al. 2017, Reprod Biol., 17(2):162-171). In vitro culture was carried out at 38.7 °C, 5% CO2, 5% O2, 90% N2, and saturated humidity. Embryos were cultured in groups until Day-6, and individually in the absence of protein (i.e. BSA or BSA+FCS) from Day-6 onwards for 24h or 40h approximately. On Day-6 (143 hours PI), excellent and good quality (grade 1 and grade 2) morulae and early blastocysts were selected and cultured individually in 12µL mSOF with 0.5 mg/mL polyvinyl-alcohol PVA (P8136, instead of BSA; instead of BSA+FCS) under mineral oil. Blastocyst development was monitored on Day-7 (168 hours PI) and Day-8 (184 hours PI).

### Embryo Vitrification, warming and in vitro survival

Vitrification procedures have been described in detail (Caamaño JN, et al. 2015. Theriogenology, 83:881-90.). Briefly, expanded blastocysts were vitrified in two-steps with fibreplugs (CryoLogic Vitrification Method; CVM). Procedures were performed on a heated surface (41 °C) in a warm room (25 °C). Embryos were handled in a basic vitrification medium (VM: TCM 199-HEPES + 20% (v/v) FCS). Groups of one to five blastocysts were exposed to VM with 7.5% ethylene-glycol (EG, 102466-M), 7.5% DMSO (D2650, vitrification solution-1) for 3 minutes, and then moved into a microdrop containing VM with 16.5% EG, 16.5% DMSO and 0.5 M sucrose (vitrification solution-2; VS2). The time spent by the embryos in VS2 (including loading) was 20 to 25 sec. Samples were vitrified by touching the surface of a supercooled block placed in LN2 with a hook. Vitrified embryos held in fibreplugs were stored in closed straws in LN2 until warming. Embryos were warmed in one step by directly immersing the fibreplug end in 800µL of 0.25 M sucrose in VM, where the embryo was kept for 5 minutes and washed twice in VM and twice in mSOF containing 6 mg/mL BSA and 10% FCS prior to ET or *in vitro* culture. In vitro survival rates were analyzed by culturing Day-7 and Day-8 vitrified embryos in droplets of 25µL of mSOF containing 6 mg/mL BSA and 10% FCS. Embryo survival was evaluated in terms of re-expansion and hatching rates at 24 and 48 hours.

### Embryo freezing and thawing

Procedures were performed on a heated surface (35°C) in a warm room (25°C). Expanded blastocysts were washed three times in PBS+4g/L BSA either individually or in groups up to 8 embryos. Subsequently, embryos were briefly washed once and loaded in freezing medium, containing PBS (P4417), 1.5M EG and 20% CRYO3 (# 5617, Stem Alpha, France) for 10 minutes. Embryos in freezing medium were aspirated in a French straw, loaded between 2 columns with PBS + 0.75M EG + 20% CRYO3, and 2 further columns PBS + 0.75M EG + 20% CRYO3 in turn separated by air (see Figure 1). The straw was closed with a plug in tight contact with a column of PBS + 0.75M EG + 20% CRYO3; this column took up approximately a half of the available length of the straw. Subsequently, straws were loaded in a programmable freezer (Crysalis, Cryocontroller PTC-9500,) at -6°C for 2 minutes and seeded once with supercooled forceps in the upper column adjacent to that contained the embryo. Straws remained for 8 further minutes at - 6°C and were subsequently dehydrated at -0.5°C/minutes up to -35°C. Ten to fifteen minutes after reaching this temperature, the straws were stored in LN2 until use. For thawing, embryos were held for 10 seconds on air, and then 30 seconds at 35 °C in a water bath. The straws were carefully dried with disposable wipes humidified with 70% ethanol. For in vitro culture, the straws were emptied in Petri dishes, making all contents to converge in a single drop. No later than 1 minute, the embryos were picked-up, washed and cultured in droplets of 25µL of mSOF containing 6 mg/mL BSA and 10% FCS. For DT to recipients, each thawed straw with a single embryo was directly mounted in an ET catheter without previous shaking or mixing contents.

### CDX2 and TUNEL staining in blastocysts

Embryos were immunohistochemically treated for simultaneous apoptotic index (TUNEL staining) and ICM and TE differential cell counts with CDX2. Hatched blastocysts (grade 1 and grade 2) surviving vitrification/warming and freezing/thawing were fixed in 4% paraformaldehyde with 0.2 mg/mL PVA and then washed and stored in phosphate buffered saline (SIGMA P4417) with 0.2 mg/mL PVA (PBS-PVA; pH=7.4, 4°C) until use. Embryos were permeabilized for 40 minutes at 37°C with sodium citrate (0.1M; pH=6.0; SIGMA C8532) containing PVA (0.2mg/mL) and Triton (1% v/v), and subsequently washed in PBS-PVA. Samples and positive controls were then submitted to TUNEL reaction according to the manufacturer's instructions (In situ Cell Death Detection Kit with Fluorescein, 11684795910, Roche®, Mannheim, BW, Germany), whereas negative controls were incubated in TUNEL mixture without transferase. Following two washes in PBS-PVA, immunohistochemical detection of CDX2 was performed. After permeabilization (15 minutes 0.5% Triton-X-100 in PBS -PVA) and washing 5 minutes in rinse buffer (RB, 0.1% Triton X-100 in PBS-PVA), samples were transferred to blocking solution (5% normal goat serum and 0.1% Triton X-100) for 2 hours at room temperature. Subsequently, samples were incubated for 72 hours at 4°C with the primary anti-CDX2 antibody (Abcam 15258) diluted in blocking solution. After washing in RB (three times 5 minutes), the embryos were incubated with Alexa Fluor conjugated secondary antibody (Goat anti-mouse Alexa Fluor®594 conjugate Thermofisher A-11032) for 1 hour and 15 minutes at RT. Finally, embryos were washed three times in RB, counterstained with DAPI and mounted on a glass slide with Vectashield-H1000 (Vector Labs, USA) under a coverslip.

Embryos were analyzed with a confocal microscope (ultra-spectral Leica TCS-SP8-AOBS; Leica Microsystems, Mannheim, Germany). An excitation wavelength of 488 nm was selected for detection of fluorescein-12-dUTP; 594 nm to excite Alexa-594; and 405 nm wavelength to excite DAPI. Photomicrographs of serial optical sections were recorded every 1.5-2 µm vertical step along the Z-axis of each embryo. CDX2 positive cells, total embryonic cells and DNA-fragmented nuclei were analyzed using software ImageJ (Confocal Uniovi ImageJ; version 1.51). Nuclei with green fluorescence (FITC) were considered TUNEL positive (fragmented DNA). Total healthy, necrotic and picnotic cells were assessed by DAPI staining of based on the presence of blue fluorescence (Gjorret et al. 2003. Biol Reprod, 69: 1193-200). Nuclei with red fluorescence were considered CDX2 positive (trophectoderm cells). Positive controls for TUNEL were carried out by treating embryos with 10 IU/mL of DNase I (Takara, Kyoto, Japan, 2215A). Negative controls for immunohistochemistry were carried out omitting the primary antibody.

### Embryo transfer, pregnancy diagnosis and calf phenotyping

Embryos were transferred in controlled conditions (experimental herd). Detailed procedures have been described (Hidalgo et al. 2004, Theriogenology, 62(3-4): 664-76.). Briefly, recipient heifers from Holstein, Asturiana de los Valles breeds and their crosses were synchronized in estrus with an intra-vaginal progestagen device (PRID Alpha; Ceva Salud Animal) for 10-11 days combined with a prostaglandin F2a analogue (Dynolitic, Pfizer,) injected 48 hours before progestagen removal. ETs were performed with fresh, vitrified/warmed and frozen/thawed Day-7 embryos. All embryos cryopreserved and transferred were expanded blastocysts, while embryos transferred fresh were early blastocysts to expanded blastocysts, always embryos that progressed to advanced stages in culture from Day-6 to Day-7. Prior to transfer, vitrified embryos were warmed and examined in their morphology; embryos with fragmented or degenerated appearance were discarded. Frozen/thawed embryos were transferred directly in straw and not examined. Fresh embryos were washed twice and mounted in straw in PBS+4g/L BSA. On Day 7 (225±1.5 hours after progestagen removal; fixed time), blastocysts were non-surgically transferred to recipients under epidural anesthesia. Pregnancy was diagnosed by ultrasonography on Day 40 and on Day 62. Birth rates were monitored. Body weights of the calf and the mother were measured at birth, as well as gestation length; average daily gain weight of the fetus was calculated. Blood plasma P4 was measured on Day 0 and Day 7 (before embryo transfer) in samples collected into ethylenediamine tetraacetic acid (EDTA) vacuum tubes via coccygeal vein puncture. An enzyme-linked immunosorbent assay (ELISA) test operating on a 0-40 ng/mL (-1) scale (DRGDiagnostics) was used. The test was sensitive starting from 0.5 ng/mL (-1) and cross-reactivity from steroids other than P4 was less than 1%. Intra and interassay coefficients of variation were 6% and 7% respectively. Recipients selected for transfers all showed rising P4 concentrations from Day-0 to Day-7 and a normal corpus luteum monitored by ultrasonography. In order to allow individual variation to express within an equilibrate design, ETs were performed in rounds (5 - 7 recipients per round), each round with embryos from a single bull (N=5 bulls) and embryos from the three treatments (i.e. V/W, F/T and fresh) whenever possible. Recipients were re-used for transfer up to three times.

### Statistics

Data requiring normalization were arcsine transformed and analyzed using the Proc GLM module of SAS/STAT (version 9.2; SAS Institute Inc., Cary, NC). In experiments concerning embryo cryopreservation and CDX2 and TUNEL staining the fixed effects included were culture up to Day-6 (i.e. BSA or FCS+BSA), embryonic stage on Day-6 (morula or blastocyst), age of the embryo (Day-7 or Day-8), and cryopreservation procedure (V/W or F/T). Replicate, bull and recipient breed were considered as random effects. Hatching time (24 or 48 hours) was analyzed as a fixed effect within CDX2 and TUNEL staining. Significant interactions between major effects were analyzed and detected. For embryo transfer and pregnancy viability (Day-40, Day-62, birth) the treatments included a group of fresh embryos transferred.

For embryo transfer, pregnancy and birth rates, the effects included were cryopreservation treatment, protein replacement up to Day-6, embryonic stage on Day-6, embryonic stage on Day-7, bull, recipient breed and ET number (1, 2, 3). For calf measurements, the effects included were protein replacement up to Day-6, cryopreservation treatment, bull, calf sex and mother weight (for normalization purposes). Bull and recipient breed were considered as random effects. Plasma P4 was analyzed using year, season, and recipient breed, as fixed effects. Calf body measurements and birth weight also included embryonic sex. Least squares means and their errors (±SEM) were estimated for each level of fixed effects with a significant F-value. The Ryan-Einot-Gabriel-Welsch Q-test was used to compare the raw means of the levels from the fixed effects (P<0.05).

### In vitro survival to cryopreservation

Table 1 shows post-cryopreservation development of embryos submitted to V/W or F/T.

**Table 1. In vitro development of bovine embryos that were cultured in vitro from Day-0 to Day-6 with 0.6%BSA or 0.6%BSA+0.1%FCS and individually, without protein replacements, from Day-6 onwards, and subjected to freezing and thawing (F/T) or vitrification and warming (V/W)**

| | | | | Live | | | 24h | | 48h | |
|---|---|---|---|---|---|---|---|---|---|---|
| Treatment | Culture to D6 | Day | N | 2h | 24h | 48h | Hatching | Hatched | Hatching | Hatched |
| F/T | BSA | 7 | 87 | 87.7±5.3**xy** | 86.5±5.5**xy** | 80.1±6.2**x** | 31.8±6.7 | 21.1±6.3 | 67.3±6.7**ab** | 56.9±6.7**ab** |
| F/T | BSA | 8 | 50 | 69.0±7.3**yz** | 67.7±7.4 | 68.0±8.5 | 7.5±9.1**c** | 0.7±8.5**a** | 43.1±9.1**bc** | 34.9±9.1**bc** |
| F/T | FCS | 7 | 87 | 78.1±6.4 | 79.5±6.5 | 71.6±7.4 | 28.2±8.0**bc** | 12.8±7.5 | 53.4±7.9**bc** | 46.4±7.9**bc** |
| F/T | FCS | 8 | 37 | 53.0±6.7**z** | 52.5+6.9**z** | 45.2±7.8**y** | 17.9±8.4 | 11.2±7.9 | 40.5±8.4**c** | 30.0±8.4**c** |
| V/W | BSA | 7 | 69 | 100.5±5.3**x** | 101.5±5.5**x** | 98.0±6.2**x** | 48.8±6.7**a** | 31.8±6.3 | 82.3±6.7**a** | 77.5±6.7a |
| V/W | BSA | 8 | 40 | 102.0±7.3**x** | 98.2±7.4**xy** | 98.3±8.5**x** | 21.2±9.1 | 23.5±8.6 | 78.9±9.1a | 70.7±9.1a |
| V/W | FCS | 7 | 60 | 105.1±6.9**x** | 106.4±7.1**x** | 103.1±8.1**x** | 53.9±8.7**ab** | 39.2±8.1**b** | 97.9±8.7a | 92.7±8.7a |
| V/W | FCS | 8 | 34 | 85.7±7.4**xy** | 84.8±7.6**xy** | 79.3±8.6**x** | 32.6±9.3 | 13.9±8.7 | 63.7±9.2**ab** | 47.9±9.2 |

| Main effects | | | | | | | | | | |
|---|---|---|---|---|---|---|---|---|---|---|
| F/T | | | 261 | 71.9±3.4* | 71.3±3.5* | 65.6±4.0* | 21.8±4.2* | 12.5±4.0* | 50.0±43* | 41.2±4.4* |
| V/W | | | 203 | 96.4±3.6 | 96.6±3.7 | 93.5±4.2 | 40.6±4.4 | 28.0±4.2 | 79.8±4.5 | 71.6±4.6 |
| | | | | | | | | | | |
| BSA | | | 246 | 88.4±3.5 | 87.2±3.5 | 84.3±4.0 | 29.5±4.3 | 20.6±4.1 | 67.0±4.4 | 58.5±4.5 |
| FCS | | | 218 | 80.4±5.0 | 80.7±5.1 | 74.8±5.8 | 32.8±6.1 | 20.0±5.8 | 62.7±6.2 | 54.2±6.4 |

| Stage on Day-6 | | | | | | | | | | |
|---|---|---|---|---|---|---|---|---|---|---|
| Blastocyst | | | 147 | 88.7±6.0 | 91.3±6.2 | 85.7±7.0 | 37.4±5.1 | 25.2±4.9 | 70.3±5.3 | 58.4±5.4 |
| Morula | | | 131 | 85.3±6.1 | 84.4±6.2 | 81.3±7.1 | 29.5±5.6 | 21.4±5.3 | 58.5±5.7 | 54.7±5.9 |
| Morula + Blastocyst | | | 186 | 91.4±5.9 | 91.6±6.1 | 87.0±6.9 | 25.1±4.8 | 14.6±4.6 | 67.7±4.9 | 58.1±5.0 |

| | | | | | | | | | | |
|---|---|---|---|---|---|---|---|---|---|---|
| *: P<0.0001; x,y: P<0.01,a,b,c: P<0.05 Data from N=11 embryo production replicates and N=6 post-cryopreservation culture replicates. Hatching: embryos with shed zona pellucida (ZP) but included in ZP Hatched: embryos outside of ZP | | | | | | | | | | |

Major effects revealed that V/W showed improved performance over F/T at all survival stages analyzed (P<0.0001). In contrast, the presence of FCS and the Day-6 stage of the embryos (morula, blastocyst or combinations of both) did not affect overall development at any stage. As usual, day-7 embryos showed higher survival rates than Day-8 (P<0.001).

Interactions between cryopreservation treatment, culture to Day-6 and age of embryos cryopreserved are shown in Table 1 (above). Within embryos subjected to F/T conditions of Day-7 and culture to Day-6 in BSA appeared to show net improved development over other F/T embryos and, interestingly, did not show significant differences at any stage with the groups of V/W embryos. Within embryos that underwent V/W, no clear interactions appeared, but embryos produced with BSA (days 7 and 8) and Day-7 embryos produced with FCS+BSA showed 100% survival at 2 hours after thawing.

### Cell Counts (CDX2 trophectodermal-cell staining) and TUNEL study

Differential cell counts were performed simultaneously with the apoptosis study in embryos that hatched after cryopreservation. Hatching time affected TE cell counts (Hatching at 24 hours: 84.0±6.4; hatching at 48 hours: 106.9±5.4; P<0.01; not shown in tables). Significant interactions are shown in Table 2, where F/T reduced the number of cells counted in the ICM of hatched embryos derived from Day-8 blastocysts. Overall, embryos undergoing F/T showed reduced cell counts in the ICM, TE and total cells.

**Table 2. Differential cell counts by CDX2 immunostaining in Day-7 and Day-8 blastocysts that hatched in culture with SOF+10%FCS after vitrification/warming or freezing/thawing. Data from N=8 post-cryopreservation culture replicates; ICM: Inner Cell Mass; TE: Trophectoderm; a,b: P<0.05**

| Cryopreservation | Day | N | ICM | TE | Total |
|---|---|---|---|---|---|
| Freezing | 7 | 55 | 29.9±1.6 | 92.3±4.1 | 122.2±5.1 |
| Freezing | 8 | 15 | 25.1±3.1a | 89.3±7.8 | 114.4±9.7 |
| Vitrification | 7 | 63 | 34.9±1.5b | 101.0±3.8 | 135.9±4.7 |
| Vitrification | 8 | 27 | 32.2±2.3b | 97.1±5.9 | 129.3±7.2 |

| Cumulative | | | | | |
|---|---|---|---|---|---|
| Freezing | | | 27.9±1.7 | 90.6±4.4 | 118.5±5.4 |
| Vitrification | | | 33.4±1.4 | 99.1±3.6 | 132.5±4.4 |
| P value | | | 0.0016 | 0.052 | 0.0099 |

In the apoptosis study, the only significant major effect was observed within supplements in culture until Day-6. Thus, the presence of 0.1% FCS + 0.6% BSA in culture, with regards to 0.6% BSA alone, increased percent apoptotic cells (P=0.018; 9.24±1.33 vs. 5.22±1.07, respectively) and tended to increase percent total dead cells (P=0.056; 16.70±2.12 vs. 11.82±1.69, respectively) (not shown in tables). Interactions in the TUNEL study were obvious only for F/T embryos with Day-7 embryos, which showed % apoptotic, % picnotic and % total dead cells significantly lower (p<0.05) than their Day-8 counterparts. These differences did not affect Day-7 vs. Day-8 embryos after V/W, although V/W did trigger picnotic and % total dead cells higher than Day-8, F/T embryos (Table 3).

**Table 3. Differential cell counts by TUNEL immunostaining in Day-7 and Day-8 blastocysts that hatched in culture with SOF+10%FCS after vitrification/warming or freezing/thawing. Embryos collected from N=8 post-cryopreservation culture replicates. a,b: P<0.05**

| Cryopreservation | Day | N | % Necrotic | % Apoptotic | % Picnotic | % Total Dead |
|---|---|---|---|---|---|---|
| Freezing | 7 | 55 | 6.06±0.71 | 8.45±1.00a | 1.62±0.32a | 16.13±1.60a |
| Freezing | 8 | 15 | 4.75±1.26 | 4.75±1.79b | 0.88±0.58b | 10.39±2.86b |
| Vitrification | 7 | 63 | 4.39±0.82 | 4.81±1.18 | 1.36±0.38a | 10.56±1.89 |
| Vitrification | 8 | 27 | 5.51±0.90 | 8.14±1.28 | 2.01±0.41a | 15.67±2.04a |

### Embryo Transfer and pregnancy monitoring

Embryos were transferred to recipients after F/T, and compared with embryos cryopreserved by a V/W system that consistently produces birth rates >45% in our laboratory. In addition, a group of fresh embryos were also transferred as negative controls. Pregnancies were monitored on Day-40 and Day-62. Results can be observed in Table 4.

**Table 4. Day-40 and Day-62 pregnancy rates after transfer of Day-7 vitrified/warmed, frozen/thawed and fresh embryos cultured from Day-0 to Day-6 in groups with either BSA (0.6%) or FCS (0.1%) + BSA (0.6%), and subsequently in individual culture without protein supplements (0.5 mg/mL PVA) from Day-6 to Day-7.**

| | Culture | | Pregnancy rates (%) | |
|---|---|---|---|---|
| Treatment | Day-0 to Day-6 | N | Day-40 | Day-62 |
| Freezing/Thawing | BSA | 24 | 14 (58) | 14 (58) |
| | FCS+BSA | 17 | 9 (53) | 8 (47) |
| Vitrification/Warming | BSA | 22 | 16 (73) | 15 (68) |
| | FCS+BSA | 11 | 5 (45) | 5 (45) |
| Fresh | BSA | 14 | 7 (50) | 7 (50) |
| | FCS+BSA | 14 | 8 (57) | 5 (36) |

| Cumulative | | | | |
|---|---|---|---|---|
| Freezing/Thawing | | 41 | 23 (56) | 22 (53) |
| Vitrification/Warming | | 33 | 21 (64) | 20 (60) |
| Fresh | | 28 | 15 (53) | 12 (43) |

| | | | | |
|---|---|---|---|---|
| No differences observed (P>0.05) (data updated July 16, 2018). | | | | |

Neither major effects nor interactions effects were observed (P>0.05) derived from cryopreservation treatments or culture to Day-6 with or without FCS. Thus, pregnancy rates with embryos undergoing F/T were similar to embryos transferred fresh and after V/W. Plasma P4 concentration increments did not change between treatments, or pregnant and open recipients according to the embryonic development pattern or protein replacement (not shown).

### Calf weight and gestation length

UntilJuly 16, 2018, calves measured were born from the first N=43 ETs that delivered N=25 calves. Data will be timely updated from pregnancies in course. Results in Table 5 show that there were not differences in birth weight, gestation length and average daily gain weight of the fetus between embryos transferred fresh and after F/T or V/W.

**Table 5. Birth weight, gestation length and average daily gain weight of calves born from Day-7 frozen/thawed, vitrified/warmed and fresh embryos cultured from Day-0 to Day-6 in groups with either BSA (0.6%) or FCS (0.1%) + BSA (0.6%), and subsequently in individual culture without protein supplements (0.5 mg/mL PVA) from Day-6 to Day-7. Birth weight values shown are both raw and normalized by mother weight at birth.**

| | | Birth weight (Kg) | | Gestation | Daily gain |
|---|---|---|---|---|---|
| | N | Raw | Normalized | length (days) | weigth (g/day) |
| Freezing/Thawing | 8 | 40.7±4.0 | 41.5±3.8 | 286.5±3.4 | 142±13 |
| Vitrification/Warming | 12 | 38.9±2.9 | 39.1±2.8 | 284.5±2.5 | 136±9 |
| Fresh | 7 | 37.2±3.4 | 38.5±3.2 | 280.1±2.8 | 132±11 |

Calves measured are born from the first N=37 ETs, which delivered N=21 calves. Other pregnancies are in course. Data are expressed as LSM±SEM. No significant differences (P>0.05) (updated 250318).

### DISCUSSION

*In vitro* survival to cryopreservation may inform of embryonic quality, sometimes not only for cryopreservation purposes, but also as a general viability test for fresh embryos. Thereby, because the high costs of recipient pregnancy management to term, experiments of embryo transfer in cattle can be preceded by analysis of embryonic viability in vitro. Anticipating pregnancy from in vitro platforms is particularly interesting within *in vitro* produced (IVP) embryos, which typically shows lower pregnancy rates, as well as high gestational losses in early pregnancy and in the late fetal and perinatal periods, morbidity and dystocia than in vivo embryos.

In the present invention the performance of a new system of embryo culture combined with F/T under chemically defined conditions for IVP embryos was analyzed. Neither cell counts nor *in vitro* survival to cryopreservation tests anticipated that, once in the uterus, frozen/thawed embryos would be able to set pregnancies at the same rates of embryos that were transferred both fresh and after V/W. In fact, once compared with their counterparts that underwent V/W, F/T embryos were significantly affected by reductions in each in vitro survival endpoint analyzed (hatching and live embryonic rates, at 24 hours and 48 hours). Furthermore, after F/T, embryos showed severe reductions in cell counts within the ICM, TE and total cells. Lastly, the TUNEL study also showed that Day-7 embryos that survived F/T showed similar proportions of dead cells that Day-8 embryos after V/W; both with the highest levels of cell damage in this experiment. However, despite of these negative survival traits observed *in vitro* with F/T, pregnancy rates, gestation length and birth weight were not affected.

One reason of the discrepancy between *in vitro* and *in vivo* experiments could lie on the presence of FCS and BSA in the post-cryopreservation culture. Vitrification solutions contain FCS, but FCS is obviously absent from chemically defined freezing media, such as the F/T medium used in the present invention. In contrast, within F/T under not chemically defined conditions (i.e., BSA containing media), expansion and hatching rates after F/T were reported to be not affected by 2 hours and 24 hours compared with V/W, and only hatching rates decreased significantly 48 hours after culture (Caamaño *et al,* 2015, cited *ad supra*). Therefore, the presence of FCS in survival culture medium could work providing a more stable environment for vitrified embryos (treated and vitrified with FCS-containing solution) than for frozen embryos (treated and frozen without FCS-containing solutions). However, defining appropriate, homogeneous post-cryopreservation survival conditions *in vitro* can be a more difficult than expected task as, to our knowledge, after embryo cryopreservation, no successful culture proceeds in the absence of serum, protein or cell-coculture has been achieved to date. Therefore, the developmental competence of embryos cryopreserved in chemically defined conditions would be generally underestimated.

Minimum numbers of cells per embryonic compartment (i.e. ICM and TE) seem to be required to set up pregnancy. Herein the quality parameters in survivor embryos after cryopreservation was tested, by which lipid granule determination was considered not necessary. It was observed that neither FCS supplementation nor Day-6 embryonic stage (i.e. morula or early blastocyst) increased apoptosis rates of hatched blastocyst after V/W. Intriguing results deal with the improved TUNEL profiles observed in Day-8 embryos subjected to F/T. Without being bound by any theory, a possible explanation for these "improvements" in the normally less viable Day-8 embryos can be the reduced numbers of ICM cells in such surviving embryos.

Both main culture conditions (i.e. with or without FCS) from Day-0 to Day-6 with all cryopreservation procedures tested, i.e. F/T, V/W and fresh embryos, were used. In the present invention, differences in pregnancy and birth rates depending on the presence of FCS within the cryopreservation system were not observed.

Here it is described an efficient chemically defined F/T system for IVP embryos, sustained by an embryo culture step without protein, replacement of BSA and/or serum by a chemical supplement (i.e. CRYO3) in cryoprotectant solutions, and a modification of in straw cryoprotectant distribution. These combinations yield high pregnancy and birth rates, comparable to those of V/W and fresh embryos. These findings provide new evidence of F/T and DT as an efficient cryopreservation system for IVP embryos, with the sanitary advantage of the absence of products of animal origin and total definition in its chemical composition.

## Claims

1. An *in vitro* method for freezing mammalian embryos, comprising
(a) culturing at least one mammalian embryo for a period between 4 and 8 days,
(b) culturing individually the mammalian embryo of step (a) in the presence of a composition which does not comprise proteins, and
(c) freezing the embryos from step (b) in liquid nitrogen in the presence of a freezing composition comprising ethylene glycol (EG), phosphate buffered saline (PBS) and synthetic cryoprotectant CRYO3.

2. Method according to claim 1, wherein the culture of the mammalian embryo in step (a) is for 6 or 7 days.

3. Method according to claim 1 or 2, wherein the mammalian embryos are those obtained by *in vivo* or *in vitro* fertilization.

4. Method according to any one of claim 1 to 3, wherein the composition of step (b) does not comprise bovine serum albumin (BSA).

5. Method according to any one of claims 1 to 4, wherein the composition for culturing the embryos in step (b) is synthetic oviduct fluid medium.

6. Method according to any one of claims 1 to 5, wherein the freezing composition comprises PBS, 1.5M EG and 20% CRYO3.

7. Method according to any one of claims 1 to 6, wherein the embryos of step (c) are loaded into a French straw between two columns comprising PBS, 0.75M EG and 20% CRYO3, and two further columns comprising PBS, 0.75M EG and 20% CRYO3 in turn separated by air.

8. An *in vitro* use of a freezing composition comprising ethylene glycol (EG), phosphate buffered saline (PBS) and synthetic cryoprotectant CRYO3 for freezing mammalian embryos cultured in the absence of serum or protein from animal origin.

9. Method according to any one of claims 1 to 8, wherein the mammalian embryo is a bovine embryo.

10. An *in vitro* method for obtaining mammalian embryos with high birth rates after embryo transfers comprising
(a) freezing an embryo from the mammal to be reproduced by a method according to any one of claims 1 to 9, and
(b) Thawing of the frozen embryos from step (a).

11. Method according to claim 10, wherein the thawing of the frozen embryos comprises exposure of the embryo-containing straw to room temperature during a time between 9 to 11 seconds and next immersing them into water at a temperature between 33 to 37°C during 28 to 32 seconds.

12. Method according to claim 11, wherein the melting of the frozen embryos comprises exposing the embryo-containing straw to room temperature during 10 seconds and next immersing them into water at 35°C during 30 seconds.

13. Method according to any one of claims 10 to 12, wherein the mammal is a bovine.
